# DEMANDE DE BREVET EUROPEEN

(11) **EP 1 944 031 A1**
(43) Date de publication de la demande: **16.07.2008**
(21) Numéro de dépôt: 08290020.0
(22) Date de dépôt: 10.01.2008
(51) Int. Cl.: A61K 31/55, A61P 9/10, A61P 25/28

(54) **Utilisation de l'ivabradine pour l'obtention de médicaments destinés au traitement de la dysfonction endotheliale**

(30) Priorité: 11.01.2007 FR 0700189
(71) Demandeur: Les Laboratoires Servier, 92415 Courbevoie Cedex (FR)
(72) Inventeur: Benatar, Vidal, 92210 Saint-Cloud (FR); Lerebours-Pigeonniere, Guy, 92300 Levallois-Perret (FR); Tardif, Jean-Claude, Laval Quebec H7E 5K2 (CA); Thorin, Eric, Monteal Quebec H1T 2C1 (CA); Rheaume, Eric, Montreal Quebec H4V 2A8 (CA)
(74) Mandataire: Giudicelli, Cathy

(57) **Abrégé**

Utilisation de l'ivabradine ou 3-{3-[{[(7S)-3,4-diméthoxybicyclo[4.2.0]octa-1,3,5-trién-7-yl]-méthyl}-(méthyl)-amino]-propyl}-7,8-diméthoxy-1,3,4,5-tétrahydro-2*H*-3-benzazépin-2-one, de ses sels d'addition à un acide pharmaceutiquement acceptable et de leurs hydrates, pour l'obtention d'un médicament destiné au traitement de la dysfonction endothéliale.

## Description

La présente invention concerne l'utilisation de l'ivabradine ou 3-{3-[{[(7S)-3,4-diméthoxybicyclo[4.2.0]octa-1,3,5-trién-7-yl]-méthyl}-(méthyl)-amino]-propyl}-7,8-diméthoxy-1,3,4,5-tétrahydro-2*H*-3-benzazépin-2-one de formule (I) : 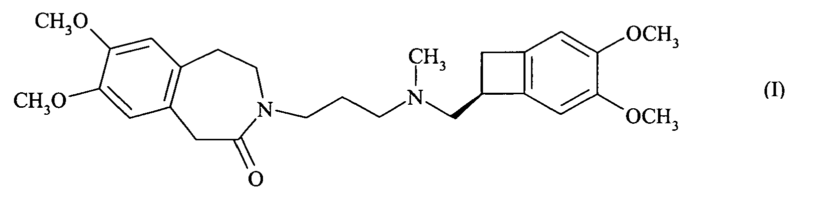 ainsi que ses sels d'addition à un acide pharmaceutiquement acceptable et les hydrates desdits sels d'addition, pour l'obtention de médicaments destinés au traitement de la dysfonction endothéliale.

L'ivabradine, ainsi que ses sels d'addition à un acide pharmaceutiquement acceptable, et plus particulièrement son chlorhydrate, et les hydrates desdits sels d'addition, possèdent des propriétés pharmacologiques et thérapeutiques très intéressantes, notamment des propriétés chronotropes négatives (réduction de la fréquence cardiaque), qui rendent ces composés utiles dans le traitement, la prévention et l'amélioration du pronostic de différentes maladies cardio-vasculaires liées à l'ischémie myocardique telles que l'angine de poitrine, l'infarctus du myocarde et les troubles du rythme associés, ainsi que dans les différentes pathologies comportant des troubles du rythme, notamment supra-ventriculaires, et dans l'insuffisance cardiaque chronique.

La préparation et l'utilisation en thérapeutique de l'ivabradine et de ses sels d'addition à un acide pharmaceutiquement acceptable, et plus particulièrement de son chlorhydrate, ont été décrits dans le brevet européen EP 0 534 859.

La demanderesse a présentement trouvé que l'ivabradine et ses sels d'addition, plus particulièrement son chlorhydrate, possédaient des propriétés intéressantes permettant leur utilisation dans le traitement de la dysfonction endothéliale.

Dans des conditions physiologiques normales, l'endothélium forme une couche semi-perméable entre les éléments circulants du sang et la paroi de tous les vaisseaux sanguins, qu'ils soient veineux ou artériels. Bien que constitué d'une seule couche de cellules, son volume total est comparable à celui du foie (Huttner et Gabbiani, Vascular endothelium in hypertension. Hypertension, éd Genest J. Kuchel O, Hamet P et Cantin M. New York: McGraw-Hill, 1983, p. 473-488) et son activité très diversifiée. En réponse à de nombreuses substances (hormones circulantes, cytokines, médicaments), à des stimuli physiques ou chimiques (force de cisaillement, changement de pression, pH), les cellules endothéliales synthétisent et libèrent divers facteurs qui modulent l'angiogénèse, les réponses inflammatoires, l'hémostase, le tonus vasculaire, la synthèse et la dégradation de la matrice extracellulaire et la perméabilité vasculaire (Félétou et Vanhoutte, Am J Physiol Heart Circ Physiol 2006, 291:985-1002).
L'une des principales substances protectrices synthétisées par l'endothélium est le monoxyde d'azote ou oxyde nitrique (NO). Le NO relaxe les cellules musculaires lisses et inhibe l'agrégation des plaquettes.

La dysfonction endothéliale est l'altération de la fonction normale des cellules endothéliales. Elle est caractérisée par une incapacité progressive des vaisseaux à s'adapter à l'environnement et à répondre aux stimuli physiologiques.

Les premiers travaux identifiant une dysfonction endothéliale datent des années 1980. Il a été observé une diminution de la relaxation endothélium-dépendante mesurée dans l'aorte de rat hypertendu (Lockette et al., Hypertension 1986, 8:1161-1166) ou chez le lapin hypercholestérolémique (Verbeuren et al., Circ Res 1986, 58:552-564). Des observations similaires ont ensuite été rapportées sur les artères coronaires de patients athérosclérotiques, suggérant que la dysfonction endothéliale pourrait être un marqueur précoce de l'athérosclérose (Ludmer et al., N. Engl. J Med 1986, 315:1046-1051).

A ce jour, elle n'est pas seulement associée à l'hypertension ou l'athérosclérose mais aussi à d'autres processus physiologiques et physiopathologiques comme l'âge, l'insuffisance cardiaque ou rénale, le syndrome coronarien, le diabète de type I et II, l'obésité, la dysfonction érectile, l'inflammation, la thrombose, le sepsis ... (Félétou et Vanhoutte, Am JPhysiol Heart Cire Physiol 2006, 291:985-1002).

Bien que la dysfonction endothéliale soit décrite dans des pathologies très variées, un dénominateur commun a été identifié : le stress oxydant. Les radicaux libres jouent un rôle central dans la physiologie et la physiopathologie vasculaire, ils sont capables notamment d'inhiber les trois voies majeures de la vasodilatation endothélium-dépendante (monoxyde d'azote, prostacycline et facteur hyperpolarisant dépendant de l'endothélium).

La demanderesse a présentement trouvé que l'ivabradine était capable de restaurer la fonction endothéliale au niveau des artères coronaires, rénales et cérébrales, chez des animaux dont la fonction endothéliale était altérée.

Cet effet permet d'envisager l'utilisation de l'ivabradine, de ses sels d'addition à un acide pharmaceutiquement acceptable et de leurs hydrates, dans le traitement de la dysfonction endothéliale, en particulier chez les patients insuffisants cardiaques, dyslipidémiques, diabétiques, hypertendus ou souffrant de syndrome métabolique, ainsi que dans la prévention, le ralentissement et le traitement de l'athérosclérose coronaire et ses complications cardiaques, de l'athérosclérose cérébrale et ses complications ischémiques et thrombotiques et de l'athérosclérose à tous les niveaux de l'arbre artériel.
De plus, l'effet bénéfique de l'ivabradine sur l'artère cérébrale (prévention de la dysfonction endothéliale et amélioration de la compliance vasculaire) permet d'envisager l'utilisation de l'ivabradine dans la neuroprotection. En effet, une dysfonction endothéliale est associée à l'ischémie cérébrale et à la maladie d'Alzheimer (Cippola et al., Stroke 2000; 31:940-945 ; Elesber et al., Neurobiology of Aging 2006; 27:446-450).

L'invention concerne donc l'utilisation de l'ivabradine, de ses sels d'addition à un acide pharmaceutiquement acceptable et de leurs hydrates, pour l'obtention de compositions pharmaceutiques destinées au traitement de la dysfonction endothéliale, en prévention de ses complications vasculaires, cardiaques et cérébrales.

Les compositions pharmaceutiques seront présentées sous des formes convenant aux administrations par voie orale, parentérale, transcutanée, nasale, rectale, perlinguale, et notamment sous forme de préparations injectables, comprimés, comprimés sublinguaux, glossettes, gélules, capsules, tablettes, suppositoires, crèmes, pommades, gels dermiques.

Parmi les compositions pharmaceutiques selon l'invention, on pourra citer plus particulièrement celles qui conviennent pour l'administration orale, parentérale, nasale, les comprimés simples ou dragéifiés, les comprimés sublinguaux, les gélules, les tablettes, les suppositoires, les crèmes, pommades, gels dermiques, ainsi que les compositions pharmaceutiques avec libération programmée, retardée, prolongée ou différée.

Outre l'ivabradine, un de ses sels d'addition à un acide pharmaceutiquement acceptable ou un des hydrates de l'ivabradine ou de l'un de ses sels d'addition, les compositions pharmaceutiques selon l'invention contiennent un ou plusieurs excipients ou véhicules tels que des diluants, des lubrifiants, des liants, des agents de désintégration, des absorbants, des colorants, des édulcorants.

A titre d'exemple et de manière non limitative, on peut citer :
◆ *pour les diluants :* le lactose, le dextrose, le sucrose, le mannitol, le sorbitol, la cellulose, la glycérine,
◆ *pour les lubrifiants* : la silice, le talc, l'acide stéarique et ses sels de magnésium et de calcium, le polyéthylène glycol,
◆ *pour les liants :* le silicate d'aluminium et de magnésium, l'amidon, la gélatine, la tragacanthe, la méthylcellulose, la carboxyméthylcellulose de sodium et la polyvinylpyrrolidone (PVP),
◆ *pour les désintégrants* : l'agar, l'acide alginique et son sel de sodium, les mélanges effervescents.

La posologie utile varie selon le sexe, l'âge et le poids du patient, la voie d'administration, la nature de l'affection et des traitements éventuellement associés et s'échelonne de 1 à 500 mg d'ivabradine par 24 heures et plus préférentiellement 10 à 15 mg par jour et de manière encore préférée de 5 à 15 mg par jour.

Le pourcentage de principe actif de formule (I) dans la composition pharmaceutique est préférentiellement compris entre 5% et 50% en poids.

### EXEMPLE 1 : Etude pharmacologique.

### Effet du chlorhydrate d'ivabradine sur la dysfonction endothéliale chez la souris dyslipidémique et le rat insuffisant cardiaque.

Par souci de simplification, "chlorhydrate d'ivabradine" a été remplacé par "Ivabradine" dans la suite de l'Exemple 1.

L'impact du traitement par l'Ivabradine sur la fonction endothéliale a été étudié dans deux modèles animaux pathologiques, des souris dyslipidémiques et des rats insuffisants cardiaques, sur 4 lits vasculaires différents, artères rénales et cérébrales chez la souris, artères coronaires et mésentériques chez les rats. L'étude de la fonction endothéliale consiste à mesurer la capacité du vaisseau à se dilater en réponse à divers stimuli tel que l'acétylcholine ou à mesurer l'augmentation du flux dans le vaisseau.
Des animaux ont été traités avec l'Ivabradine, 10 mg/kg par jour pendant 3 mois. Des souris dyslipidémiques, des rats insuffisants cardiaques, des souris saines ("Wild type") et des rats sains non traités servaient de contrôle. A la fin de la période de traitement, les artères étaient isolées et montées dans un myographe afin de mesurer leur diamètre suite à l'application de l'acétylcholine ou de l'augmentation du flux. Dans le but d'explorer plus finement le mécanisme associé à la dysfonction endothéliale, des inhibiteurs des principales voies de vasodilatation ont été testés, un chélateur de radicaux libres, un inhibiteur de la production de monoxyde d'azote, un inhibiteur de la voie de la prostacycline.
Enfin, la compliance (capacité du vaisseau à se dilater avec l'accroissement de la pression transmurale) des artères cérébrales des souris a aussi été évaluée.

### Résultats

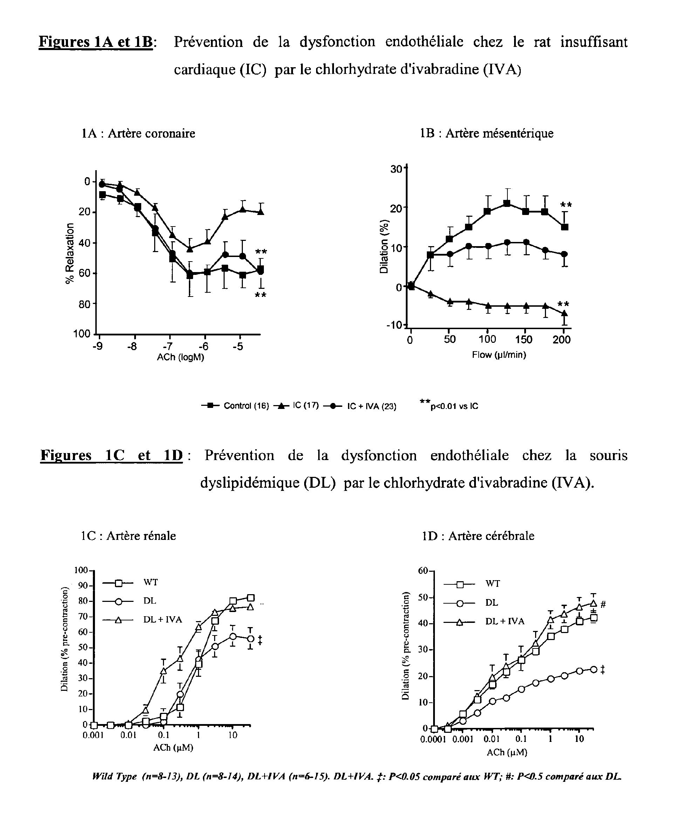

Chez les rats insuffisants cardiaques (IC) et les souris dyslipidémiques (DL), il est observé une dysfonction endothéliale dans tous les vaisseaux étudiés. La capacité de ces vaisseaux à se dilater est diminuée, d'environ 20% par rapport aux animaux sains, dans l'artère coronaire, rénale et cérébrale et elle est nulle dans l'artère mésentérique du rat IC (Figure 1).

Après 3 mois de traitement, l'Ivabradine induit une baisse significative (10-20%) de la fréquence cardiaque. L'Ivabradine prévient complètement la dysfonction endothéliale au niveau des artères coronaires, rénales et cérébrales. La dilatation mesurée dans ces artères est similaire à celle mesurée dans les artères issues des animaux sains (Figure 1A, C, D). La capacité à se dilater de l'artère mésentérique du rat IC traité (Figure 1B) est nettement améliorée par rapport à l'artère issue du rat IC.

Les mécanismes impliqués dans ces effets bénéfiques de l'Ivabradine sont une diminution du stress oxydant et une préservation de la voie du monoxyde d'azote.

Enfin, la compliance des artères cérébrales isolées de souris traitées est améliorée.

### EXEMPLE 2 : Composition pharmaceutique

Formule de préparation pour 1000 comprimés dosés à 5 mg d'ivabradine base:

| | |
|---|---|
| Chlorhydrate d'ivabradine | 5,39 g |
| Amidon de maïs | 20 g |
| Silice colloïdale anhydre | 0,2 g |
| Mannitol | 63,91 g |
| PVP | 10 g |
| Stéarate de magnésium | 0,5 g |

## Revendications

1. Utilisation de l'ivabradine ou 3-{3-[{[(7S)-3,4-diméthoxybicyclo[4.2.0]octa-1,3,5-trién-7-yl]-méthyl}-(méthyl)-amino]-propyl}-7,8-diméthoxy-1,3,4,5-tétrahydro-2*H*-3-benzazépin-2-one, de ses sels d'addition à un acide pharmaceutiquement acceptable et de leurs hydrates, pour l'obtention d'un médicament destiné au traitement de la dysfonction endothéliale.

2. Utilisation selon la revendication 1 pour l'obtention d'un médicament destiné au traitement de la dysfonction endothéliale chez les patients insuffisants cardiaques, dyslipidémiques, diabétiques, hypertendus ou souffrant de syndrome métabolique.

3. Utilisation selon la revendication 1 pour l'obtention d'un médicament destiné à la prévention, ralentissement ou au traitement de l'athérosclérose.

4. Utilisation selon la revendication 1 pour l'obtention d'un médicament destiné à la neuroprotection.

5. Utilisation selon la revendication 1 pour l'obtention d'un médicament destiné au traitement de l'ischémie cérébrale ou de la maladie d'Alzheimer.

6. Composition pharmaceutique contenant de l'ivabradine ou 3-{3-[{[(7S)-3,4-diméthoxybicyclo[4.2.0]octa-1,3,5-trién-7-yl]-méthyl}-(méthyl)-amino]-propyl}-7,8-diméthoxy-1,3,4,5-tétrahydro-2*H*-3-benzazépin-2-one, de ses sels d'addition à un acide pharmaceutiquement acceptable et de leurs hydrates, en combinaison avec un ou plusieurs excipients pharmaceutiquement acceptables, utile pour le traitement de la dysfonction endothéliale.

7. Composition pharmaceutique selon la revendication 6, utile pour le traitement de la dysfonction endothéliale chez les patients insuffisants cardiaques, dyslipidémiques, diabétiques, hypertendus ou souffrant de syndrome métabolique.

8. Composition pharmaceutique selon la revendication 6, utile pour la prévention, le ralentissement ou le traitement de l'athérosclérose.

9. Composition pharmaceutique selon la revendication 6, utile pour la neuroprotection.

10. Composition pharmaceutique selon la revendication 6, utile pour le traitement de l'ischémie cérébrale ou de la maladie d'Alzheimer.
